# EUROPÄISCHE PATENTSCHRIFT

(11) **EP 2 035 415 B1**
(45) Veröffentlichungstag und Bekanntmachung des Hinweises auf die Patenterteilung: **12.01.2011**
(21) Anmeldenummer: 07765511.6
(22) Anmeldetag: 20.06.2007
(51) Int. Cl.: C07D 405/06, C07D 405/14, C07D 409/14, A01N 43/653

(54) **AZOLYLMETHYLOXIRANE, IHRE VERWENDUNG ZUR BEKÄMPFUNG VON PFLANZENPATHOGENEN PILZEN SOWIE SIE ENTHALTENDE MITTEL**
AZOLYLMETHYLOXIRANES, THEIR USE FOR CONTROLLING PHYTOPATHOGENIC FUNGI AND COMPOSITIONS COMPRISING THEM
AZOLYLMÉTHYLOXIRANE, SON UTILISATION POUR LUTTER CONTRE LES CHAMPIGNONS PATHOGÈNES DES PLANTES ET AGENTS CONTENANT CE COMPOSÉ

(30) Priorität: 23.06.2006 EP 06115936
(43) Veröffentlichungstag der Anmeldung: 18.03.2009
(73) Patentinhaber: BASF SE, 67056 Ludwigshafen (DE)
(72) Erfinder: DIETZ, Jochen, 68167 Mannheim (DE); GROTE, Thomas, 67157 Wachenheim (DE); MÜLLER, Bernd, 67227 Frankenthal (DE); LOHMANN, Jan Klaas, 67063 Ludwigshafen (DE); RENNER, Jens, 67098 Bad Dürkheim (DE); ULMSCHNEIDER, Sarah, 67098 Bad Dürkheim (DE); GLÄTTLI, Alice, 68165 Mannheim (DE)
(86) Internationale Anmeldenummer: PCT/EP2007/056124
(87) Internationale Veröffentlichungsnummer: WO 2007/147841

(56) Entgegenhaltungen:
- EP-A- 0 421 125
- EP-A2- 0 196 038

## Beschreibung

Die vorliegende Erfindung betrifft Azolylmethyloxlrane der allgemeinen Formel I 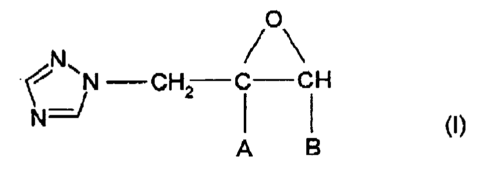 worin
- A: für Phenyl steht, das mit einem F und einem weiteren Substituenten substituiert ist, ausgewählt aus Cl, C₁-C₄-Alkyl, C₁-C₄-Halogenalkyl und C₁-C₄-Alkoxy, wobei der Phenylring in 2,4-Stellung substituiert ist,
- B: für Phenyl steht, das durch ein bis drei der folgenden Substituenten Halogen, NO₂, Amino, C₁-C₄-Alkoxy, C₁-C₄-Halogenalkyl, C₁-C₄-Halogenalkoxy, C₁-C₄- Alkylamino, C₁-C₄-Dialkylamino, Thio oder C₁-C₄-Alkylthio, substituiert ist,
sowie deren für Pflanzen verträgliche Säureadditions- oder Metallsalze.

Weiterhin betrifft die Erfindung die Verwendung der Verbindungen der Formel I zur Bekämpfung von pflanzenpathogenen Pilzen und sie enthaltende Mittel.

Azolylmethyloxirane, ihre Herstellung und ihre Verwendung im Pflanzenschutz sind beispielsweise aus der EP-A 0 094 564 und der EP-A 0 196 038 bekannt.

Aus der EP-A 0 421 125 sind Azolylmethyloxirane bekannt, die am Oxiran-Ring einen Hetaryl-Substituenten tragen.

Obwohl die beschriebenen Azolylmethyloxlrane bereits eine gute bis sehr gute fungizide Wirkung gegen eine Reihe von Pathogenen aufweisen, lag der vorliegenden Erfindung als Aufgabe zugrunde, neue Azolylmethyloxirane mit einer verbesserten fungiziden Wirkung zur Verfügung zu stellen.

Diese Aufgabe wurde mit den eingangs beschriebenen Verbindungen der Formel I gelöst.

Die Verbindung I ist wegen des basischen Charakters der in ihnen enthaltenen Stickstoffatome in der Lage, mit anorganischen oder organischen Säuren oder mit Metallionen Salze oder Addukte zu bilden.

Beispiele für anorganische Säuren sind Halogenwasserstoffsäuren wie Fluorwasserstoff, Chlorwasserstoff, Bromwasserstoff und Jodwasserstoff, Kohlensäure, Schwefelsäure, Phosphorsäure und Salpetersäure.

Als organischen Säuren kommen beispielsweise Ameisensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche eine oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche eine oder zwei Phosphorsäurereste tragen), wobei die Alkyl- bzw. Arylreste weitere Substituenten tragen können, z.B. p-Toluolsulfonsäure, Salizylsäure, p-Aminosalizylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der Nebengruppen der vierten Periode. Die Metalle können dabei in den verschiedenen ihnen zukommenden Wertigkeiten vorliegen.

Die Herstellung der Verbindungen der Formel I ist bekannt und in den EP-A 0 094 564, EP-A 0 196 038 und EP-A 0 421 125 ausführlich beschrieben.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl sowie die Alkylteile von zusammengesetzten Gruppen wie beispielsweise Alkylamino: gesättigte, geradkettige oder verzweigte Kohlenwasserstoffreste mit vorzugsweise 1 bis 4 Kohlenstoffatomen, wie Methyl, Ethyl, Propyl, 1-Methylethyl, Butyl, 1-Methyl-propyl, 2-Methylpropyl und 1,1-Dimethylethyl.
Halogenalkyl: Alkyl wie vorstehend genannt, wobei in diesen Gruppen teilweise oder vollständig die Wasserstoffatome durch Halogenatome wie vorstehend genannt ersetzt sind. In einer Ausführungsform sind die Alkylgruppen mindestens ein Mal oder vollständig durch ein bestimmtes Halogenatom substituiert, vorzugsweise Fluor, Chlor oder Brom. In einer weiteren Ausführungsform sind die Alkylgruppen durch verschiedene Halogenatome teilweise oder vollständig halogeniert; bei gemischten Halogensubstitutionen ist die Kombination Chlor und Fluor bevorzugt. Insbesondere bevorzugt sind (C₁-C₄)-Halogenalkyl, mehr bevorzugt (C₁-C₂)-Halogenalkyl, wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
Alkoxy: für eine über ein Sauerstoff gebundene Alkylgruppe wie oben definiert, bevorzugt mit 1 bis 4 C-Atomen. Beispiele für bevorzugte Alkoxygruppen sind: Methoxy, E-thoxy, n-Propoxy, 1-Methylethoxy, Butoxy, 1-Methylpropoxy, 2-Methylpropoxy oder 1,1-Dimethylethoxy.
Halogenalkoxy: Alkoxy, wie vorstehend definiert, wobei in diesen Gruppen die Wasserstoffatome teilweise oder vollständig gegen Halogenatome, wie vorstehend unter Halogenalkyl beschrieben, insbesondere Fluor, Chlor oder Brom, ersetzt sind. Beispiele für bevorzugte Halogenalkoxyreste sind OCH₂F, OCHF₂, OCF₃, OCH₂Cl, OCHCl₂, OCCl₃, Chlorfluormethoxy, Dichlorfluormethoxy, Chlordifluormethoxy, 2-Fluorethoxy, 2-Chlorethoxy, 2-Bromethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy.
Alkylthio: Alkyl, wie vorstehend definiert, das über ein S-Atom gebunden ist.

Die neuen Verbindungen der Formel I enthalten chirale Zentren und werden im allgemeinen in Form von Racematen oder als Diastereomerengemische von erythro- sowie threo-Formen erhalten. Die erythro- und threo-Diastereomeren lassen sich bei den erfindungsgemäßen Verbindungen beispielsweise aufgrund ihrer unterschiedlichen Löslichkeit oder durch Säulenchromatographie trennen und in reiner Form isolieren. Aus solchen einheitlichen Diastereomerenpaaren kann man mit bekannten Methoden einheitliche Enantiomere erhalten. Als antimikrobielle Mittel kann man sowohl die einheitlichen Diastereomere bzw. Enantiomere wie auch deren bei der Synthese anfallende Gemische verwenden. Entsprechendes gilt für die fungiziden Mittel. Bevorzugt sind hierbei Enantiomerenpaare oder Enantiomere mit *cis*-Anordnung von Ring B und Triazolylmethylsubstituent.

Die erfindungsgemäßen Verbindungen können in verschiedenen Kristallmodifikationen vorliegen, die sich in der biologischen Wirksamkeit unterscheiden können. Sie sind ebenfalls Gegenstand der vorliegenden Erfindung.

In den erfindungsgemäßen bzw. erfindungsgemäß verwendeten Verbindungen der Formel I sind die folgenden Bedeutungen der Substituenten, und zwar jeweils für sich allein oder in Kombination, besonders bevorzugt. Die bevorzugten Substituenten oder bevorzugten Kombinationen von Substituenten gelten dabei ggf. entsprechend für die Vorstufen der erfindungsgemäßen Verbindungen.

Der Substituent A steht für Phenyl, das mit einem F und einem weiteren Substituenten, ausgewählt aus Cl, C₁-C₄-Alkyl, C₁-C₄-Halogenalkyl und C₁-C₄-Alkoxy substituiert ist, wobei der Phenylring in 2,4-Stellung substituiert.

Gemäß einer weiteren Ausführungsform steht A für Phenyl, das mit einem F und einem weiteren Substituenten ausgewählt aus Cl, Methyl, Trifluormethyl und Methoxy substituiert ist.

In einer bevorzugten Ausführungsform steht der Substituent A für Phenyl, das mit einem F und einem weiteren Substituenten, ausgewählt aus Methyl, Methoxy und Chlor substituiert ist, so dass sich insgesamt 12 Substituenten A1 bis A12 der folgenden Formeln ergeben:

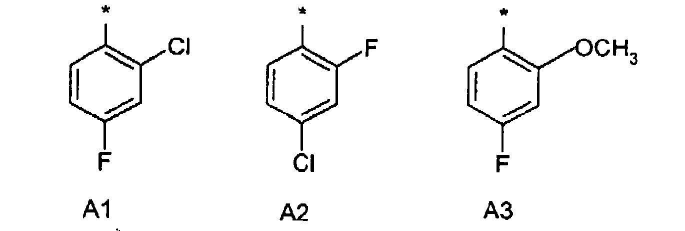

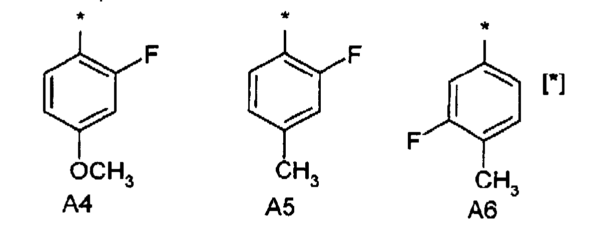

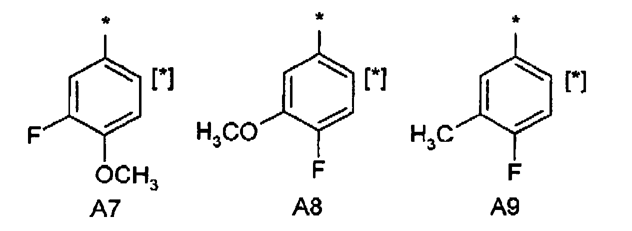

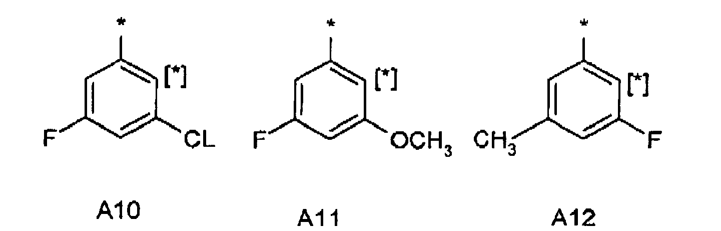

Gemäß einer Ausführungsform steht A für A1 bis A5.

Gemäß einer weiteren Ausführungsform steht A für A1, A2 oder A3.

Gemäß einer bevorzugten Ausführungsform steht A für A1 oder A2.

Gemäß einer weiteren Ausführungsform steht A für A1.

Gemäß einer weiteren Ausführungsform steht A für A2.

Gemäß einer weiteren Ausführungsform steht A für A3.

[*] = nicht vom Schutzumfang umfasst

Gemäß einer weiteren Ausführungsform steht A für A4.

Gemäß einer weiteren Ausführungsform steht A für A5.

Gemäß einer weiteren Ausführungsform steht A für A6, A7, A8 oder A9.

Gemäß einer weiteren Ausführungsform steht A für A6, A8 oder A9.

Gemäß einer bevorzugten Ausführungsform steht A für A8 oder A9.

Gemäß einer besonders bevorzugten Ausführungsform steht A für A9.

Gemäß einer weiteren Ausführungsform steht A für A6.

Gemäß einer weiteren Ausführungsform steht A für A7.

Gemäß einer weiteren Ausführungsform steht A für A8.

Gemäß einer weiteren Ausführungsform steht A für A10, A11 oder A12.

Gemäß einer besonders bevorzugten Ausführungsform steht A für A10.

Gemäß einer weiteren Ausführungsform steht A für A11.

Gemäß einer weiteren Ausführungsform steht A für A12.

In einer weiteren Ausführungsform der vorliegenden Erfindung steht B für Phenyl, das durch ein bis drei der folgenden Substituenten Halogen, NO₂, Amino, C₁-C₄-Alkoxy, C₁-C₄-Halogenalkyl, C₁-C₄-Halogenalkoxy, C₁-C₄-Alkylamino, C₁-C₄-Dialkylamino, Thio oder C₁-C₄-Alkylthio substituiert ist.

In einer weiteren Ausführungsform steht B für Phenyl, das durch ein bis drei der folgenden Substituenten Halogen, C₁-C₄-Alkoxy, C₁-C₄-Halogenalkyl oder C₁-C₄-Halogenalkoxy substituiert ist.

In einer bevorzugten Ausführungsform steht B für Phenyl, das durch ein bis drei Halogen substituiert ist. Insbesondere sind im Hinblick auf ihre Verwendung die in den folgenden Tabellen 2 bis 13 zusammengestellten erfindungsgemäßen Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.
[*] = nicht vom Schutzumfang umfasst

**Tabelle 1**

| **Zeile** | **Substituent B** |
|---|---|
| 1-1 | 2-Methylphenyl [*] |
| 1-2 | 3-Methylphenyl [*] |
| 1-3 | 4-Methylphenyl [*] |
| 1-4 | 2-Methoxyphenyl |
| 1-5 | 3-Methoxyphenyl |
| 1-6 | 4-Methoxyphenyl |
| 1-7 | 2-Chlorphenyl |
| 1-8 | 3-Chlorphenyl |
| 1-9 | 4-Chlorphenyl |
| 1-10 | 2-Fluorpnenyl |
| 1-11 | 3-Fluorphenyl |
| 1-12 | 4-Fluorphenyl |
| 1-13 | 2-Chlor-3-Methoxyphenyl |
| 1-14 | 2-Chlor-4-Methoxyphenyl |
| 1-15 | 2,3-Dichlorphenyl |
| 1-16 | 2,4-Dichlorphenyl |
| 1-17 | 3,4-Dichlorphenyl |
| 1-18 | 2,3-Difluorphenyl |
| 1-19 | 2,4-Difluorphenyl |
| 1-20 | 2-Chlor-3-Fluorphenyl |
| 1-21 | 2-Chlor-4-Fluorphenyl |
| 1-22 | 2-Pyridyl [*] |
| 1-23 | 3-Pyridyl [*] |
| 1-24 | 4-Pyridyl [*] |
| 1-25 | 2-Thienyl [*] |
| 1-26 | 3-Thienyl [*] |
| 1-27 | Thiazol-4-yl [*] |
| 1-28 | Tniazol-5-yl [*] |
| 1-29 | Oxazol-4-yl [*] |
| 1-30 | Oxazol-5-yl [*] |
| 1-31 | 2-Furyl [*] |

### Tabelle 2

Verbindungen der Formel I, in denen A für A1 steht und B jeweils einem Substituenten einer Zeile der Tabelle 1 entspricht, wobei 2-Methylphenyl ausgenommen ist.

### Tabelle 3

Verbindungen der Formel I, in denen A für A2 steht und B jeweils einem Substituenten einer Zeile der Tabelle 1 entspricht.

### Tabelle 4

Verbindungen der Formel I, in denen A für A3 steht und B jeweils einem Substituenten einer Zeile der Tabelle 1 entspricht.

### Tabelle 5

Verbindungen der Formel I, in denen A für A4 steht und B jeweils einem Substituenten einer Zeile der Tabelle 1 entspricht.

### Tabelle 6

Verbindungen der Formel I, in denen A für A5 steht und B jeweils einem Substituenten einer Zeile der Tabelle 1 entspricht.

### Tabelle 7

Verbindungen der Formel I, in denen A für A6 steht und B jeweils einem Substituenten einer Zeile der Tabelle 1 entspricht.

### Tabelle 8

Verbindungen der Formel I, in denen A für A7 steht und B jeweils einem Substituenten einer Zeile der Tabelle 1 entspricht.

### Tabelle 9

Verbindungen der Formel I, in denen A für A8 steht und B jeweils einem Substituenten einer Zeile der Tabelle 1 entspricht.

### Tabelle 10

Verbindungen der Formel I, in denen A für A9 steht und B jeweils einem Substituenten einer Zeile der Tabelle 1 entspricht.

### Tabelle 11

Verbindungen der Formel I, in denen A für A10 steht und B jeweils einem Substituenten einer Zeile der Tabelle 1 entspricht.

### Tabelle 12

Verbindungen der Formel I, in denen A für A11 steht und B jeweils einem Substituenten einer Zeile der Tabelle 1 entspricht.

### Tabelle 13

Verbindungen der Formel I, in denen A für A12 steht und B jeweils einem Substituenten einer Zeile der Tabelle 1 entspricht.

Die Verbindungen I eignen sich als Fungizide. Sie zeichnen sich aus durch eine hervorragende Wirksamkeit gegen ein breites Spektrum von pflanzenpathogenen Pilzen aus der Klasse der Ascomyceten, Deuteromyceten, Oomyceten und Basidiomyceten, insbesondere aus der Klasse der Oomyceten. Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen.

Speziell eignen sie sich zur Bekämpfung folgender Pflanzenkrankheiten:
- Alternaria Arten an Gemüse, Raps, Zuckerrüben und Obst und Reis , wie z.B. A.solani oder A. alternata an Kartoffeln und Tomaten,
- Aphanomyces Arten an Zuckerrüben und Gemüse,
- Ascochyta-Arten an Getreide and Gemüse,
- Bipolaris- und Drechslera Arten an Mais, Getreide, Reis und Rasen, wie z.B. D.maydis an Mais,
- Blumeria graminis (Echter Mehltau) an Getreide,
- Botrytis cinerea (Grauschimmel) an Erdbeeren, Gemüse, Blumen und Weinreben,
- Bremia lactucae an Salat,
- Cercospora Arten an Mais, Sojabohnen, Reis und Zuckerrüben,
- Cochliobolus Arten an Mais , Getreide, Reis, wie z.B. Cochliobolus sativus an Getreide, Cochliobolus miyabeanus an Reis,
- Colletotricum Arten an Sojabohnen und Baumwolle,
- Drechslera Arten, Pyrenophora Arten an Mais, Getreide, Reis und Rasen, wie z.B. D.teres an Gerste oder D. tritici-repentis an Weizen,
- Esca an Weinrebe, verursacht durch Phaeoacremonium chlamydosporium, Ph. Aleophilum, und Formitipora punctata (syn. Phellinus punctatus),
- Exserohilum Arten an Mais,
- Erysiphe cichoracearum und Sphaerotheca fuliginea an Gurkengewächsen,
- Fusarium und Verticillium Arten an verschiedenen Pflanzen wie z.B. F. graminearum oder F. culmorum an Getreide oder F. oxysporum an einer Vielzahl von Pflanzen wie z.B. Tomaten,
- Gaeumanomyces graminis an Getreide,
- Gibberella arten an Getreide und Reis (z.B. Gibberella fujikuroi an Reis),
- Grainstaining complex an Reis,
- Helminthosporium Arten an Mais und Reis,
- Michrodochium nivale an Getreide,
- Mycosphaerella Arten an Getreide, Bananen und Erdnüssen, wie z.B. M. graminicola an Weizen oder M.fijiensis an Bananen,
- Peronospora-Arten an Kohl und Zwiebelgewächsen, wie z.B. P. brassicae an Kohl oder P. destructor an Zwiebel,
- Phakopsara pachyrhizi und Phakopsara meibomiae an Sojabohnen,
- Phomopsis Arten an Sojabohnen und Sonnenblumen,
- Phytophthora infestans an Kartoffeln und Tomaten,
- Phytophthora Arten an verschiedenen Pflanzen wie z.B. P.capsici an Paprika,
- Plasmopara viticola an Weinreben,
- Podosphaera leucotricha an Apfel,
- Pseudocercosporella herpotrichoides an Getreide,
- Pseudoperonospora an verschiedenen Pflanzen wie z.B. P. cubensis an Gurke oder P. humili an Hopfen,
- Puccinia Arten an verschiedenen Pflanzen wie z.B. P. triticina, P. striformins, P. hordei oder P.graminis an Getreide, oder P. asparagi an Spargel,
- Pyricularia oryzae , Corticium sasakii , Sarocladium oryzae, S.attenuatum, Entyloma oryzae, an Reis,
- Pyricularia grisea an Rasen und Getreide,
- Pythium spp. an Rasen, Reis, Mais, Baumwolle, Raps, Sonnenblumen, Zuckerrüben, Gemüse und anderen Pflanzen wie z.B. P.ultiumum an verschiedenen Pflanzen, P. aphanidermatum an Rasen,
- Rhizoctonia-Arten an Baumwolle, Reis, Kartoffeln, Rasen, Mais, Raps, Kartoffeln, Zuckerrüben, Gemüse und an verschiedenen Pflanzen wie z.B. R.solani an Rüben und verschiedenen Pflanzen,
- Rhynchosporium secalis an Gerste, Roggen und Triticale,
- Sclerotinia Arten an Raps und Sonnenblumen,
- Septoria tritici und Stagonospora nodorum an Weizen,
- Erysiphe (syn. Uncinula) necator an Weinrebe,
- Setospaeria Arten an Mais und Rasen,
- Sphacelotheca reilinia an Mais,
- Thievaliopsis Arten an Sojabohnen und Baumwolle,
- Tilletia Arten an Getreide,
- Ustilago-Arten an Getreide, Mais und Zuckerrohr, wie z.B. U. maydis an Mais,
- Venturia-Arten (Schorf) an Äpfeln und Birnen wie. z.B. V. inaequalis an Apfel.

Insbesondere eignen sie sich zur Bekämpfung von Schadpilzen aus der Klasse der Peronosporomycetes (syn.Oomyceten), wie Peronospora-Arten, Phytophthora-Arten, Plasmopara viticola , Pseudoperonospora-Arten und Pythium-Arten.

Die Verbindungen I eignen sich außerdem zur Bekämpfung von Schadpilzen im Materialschutz (z.B. Holz, Papier, Dispersionen für den Anstrich, Fasern bzw. Gewebe) und im Vorratsschutz. Im Holzschutz finden insbesondere folgende Schadpilze Beachtung: Ascomyceten wie Ophiostoma spp., Ceratocystis spp., Aureobasidium pullulans, Sclerophoma spp., Chaetomium spp., Humicola spp., Petriella spp., Trichurus spp.; Basidiomyceten wie Coniophora spp., Coriolus spp., Gloeophyllum spp., Lentinus spp., Pleurotus spp., Poria spp., Serpula spp. und Tyromyces spp., Deuteromyceten wie Aspergillus spp., Cladosporium spp., Penicillium spp., Trichoderma spp., Alternaria spp., Paecilomyces spp. und Zygomyceten wie Mucor spp., darüber hinaus im Materialschutz folgende Hefepilze: Candida spp. und Saccharomyces cerevisae.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 1 bis 1000 g/100 kg, vorzugsweise 5 bis 100 g/100 kg Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen der Formel I können in verschiedenen Kristallmodifikationen vorliegen, die sich in der biologischen Wirksamkeit unterscheiden können. Sie sind ebenfalls Gegenstand der vorliegenden Erfindung.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden. Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

### Beispiele für Formulierungen sind: 1. Produkte zur Verdünnung in Wasser

### A Wasserlösliche Konzentrate (SL, LS)

10 Gew.-Teile der Wirkstoffe werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate (DC)

20 Gew.-Teile der Wirkstoffe werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%

### C Emulgierbare Konzentrate (EC)

15 Gew.-Teile der Wirkstoffe werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen (EW, EO, ES)

25 Gew.-Teile der Wirkstoffe werden in 35 Gew.-Teile Xylol unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen (SC, OD, FS)

20 Gew.-Teile der Wirkstoffe werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-% .

### F Wasserdispergierbare und wasserlösliche Granulate (WG, SG)

50 Gew.-Teile der Wirkstoffe werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

### G Wasserdispergierbare und wasserlösliche Pulver (WP, SP, SS, WS)

75 Gew.-Teile der Wirkstoffe werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen (GF)

In einer Kugelmühle werden 20 Gew.-Teile der Wirkstoffe, 10 Gew.-Teile Dispergiermittel, 1Gew.-Teil Quellmittel ("gelling agent") und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

### I Stäube (DP, DS)

5 Gew.-Teile der Wirkstoffe werden fein gemahlen und mit 95 Gew.-Teilen feinteiligem Kaolin innig vermischt. Man erhält dadurch ein Stäubemittel mit 5 Gew.-% Wirkstoffgehalt.

### J Granulate (GR, FG, GG, MG)

0,5 Gew-Teile der Wirkstoffe werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

### K ULV- Lösungen (UL)

10 Gew.-Teile der Wirkstoffe werden in 90 Gew.-Teilen eines organischen Lösungsmittel z.B. Xylol gelöst. Dadurch erhält man ein Produkt für die Direktapplikation mit 10 Gew.-% Wirkstoffgehalt.

Für die Saatgutbehandlung werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gelformulierungen (GF) verwendet. Diese Formulierungen können auf das Saatgut unverdünnt oder, bevorzugt, verdünnt angewendet werden. Die Anwendung kann vor der Aussaat erfolgen.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:100 bis 100:1, bevorzugt 1:10 bis 10:1 zugemischt werden.

Als Adjuvants in diesem Sinne kommen insbesondere in Frage: organisch modifizierte Polysiloxane, z.B. Break Thru S 240®; Alkoholalkoxylate, z. B. Atplus 245®, Atplus MBA 1303®, Plurafac LF 300® und Lutensol ON 30®; EO-PO-Blockpolymerisate, z. B. Pluronic RPE 2035® und Genapol B®; Alkoholethoxylate, z. B. Lutensol XP 80®; und Natriumdioctylsulfosuccinat, z. B. Leophen RA®.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel mit einem oder mehreren weiteren Wirkstoffen, insbesondere Fungiziden, kann beispielsweise in vielen Fällen das Wirkungsspektrum verbreitert werden oder Resistenzentwicklungen vorgebeugt werden. In vielen Fällen erhält man dabei synergistische Effekte.

Ein weiterer Gegenstand der vorliegenden Erfindung ist eine Kombination aus mindestens einem Azolylmethyloxiran der Formel I, insbesondere einem in der vorliegenden Beschreibung als bevorzugt offenbartes Azolylmethyloxiran und/oder einem landwirtschaftlich verträglichen Salz davon und mindestens einem weiteren fungiziden, insektiziden, herbiziden und/oder wachstumsregulierenden Wirkstoff, wobei eine synergistische Wirkung auftreten kann.

Noch ein Gegenstand der vorliegenden Erfindung ist ein pestizides Mittel, umfassend mindestens eine Verbindung der Formel I, insbesondere eine in der vorliegenden Beschreibung als bevorzugt beschriebene Verbindung der Formel I und/oder ein landwirtschaftlich verträgliches Säureadditions- oder Metallsalz davon und mindestens einen festen oder flüssigen Trägerstoff. Ein solches pestizides Mittel kann mindestens einen weiteren fungiziden, insektiziden und/oder herbiziden Wirkstoff enthalten, wobei auch eine synergistische Wirkung auftreten kann.

Die folgende Liste L von Fungiziden, mit denen die erfindungsgemäßen Verbindungen gemeinsam angewendet werden können, soll die Kombinationsmöglichkeiten erläutern, nicht aber einschränken:

### Liste L:

### Strobilurine

Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethyl ester, 2-(ortho-(2,5-Dimethylphenyl-oxymethylen)phenyl)-3-methoxy-acrylsäuremethylester; L-2

### Carbonsäureamide

- Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3,4-Dichlor-isothiazol-5-carbonsäure-(2-cyano-phenyl)-amid;
- Carbonsäuremorpholide: Dimethomorph, Flumorph;
- Benzoesäureamide: Flumetover, Fluopicolide (Picobenzamid), Zoxamide;
- Sonstige Carbonsäureamide: Carpropamid, Diclocymet, Mandipropamid, N-(2-(4-[3-(4-Chlor-phenyl)-prop-2-inyloxy]-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid;

### Azole

- Triazole: Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, Ipconazole, Metconazol, Myclobutanil, Penconazole, Propiconazole, Prothioconazole, Simeconazole, Tebuconazole, Tetraconazole, Triadimenol, Triadimefon, Triticonazole;
- Imidazole: Cyazofamid, Imazalil, Pefurazoate, Prochloraz, Triflumizole;
- Benzimidazole: Benomyl, Carbendazim, Fuberidazole, Thiabendazole;
- Sonstige: Ethaboxam, Etridiazole, Hymexazole;

### Stickstoffhaltige Heterocyclylverbindungen

- Pyridine: Fluazinam, Pyrifenox, 3-[5-(4-Chlor-phenyl)-2,3-dimethyl-isoxazolidin-3-yl]-pyridin;
- Pyrimidine: Bupirimate, Cyprodinil, Ferimzone, Fenarimol, Mepanipyrim, Nuarimol, Pyrimethanil;
- Piperazine: Triforine;
- Pyrrole: Fludioxonil, Fenpiclonil;
- Morpholine: Aldimorph, Dodemorph, Fenpropimorph, Tridemorph;
- Dicarboximide: Iprodione, Procymidone, Vinclozolin;
- sonstige: Acibenzolar-S-methyl, Anilazin, Captan, Captafol, Dazomet, Diclomezine, Fenoxanil, Folpet, Fenpropidin, Famoxadone, Fenamidone, Octhilinone, Probenazole, Proquinazid, Pyroquilon, Quinoxyfen, Tricyclazole, 5-Chlor-7-(4-methyl-piperidin-1-yl)-6-(2,4,6-trifluor-phenyl)-[1,2,4]triazolo[1,5-a]pyrimidin, 2-Butoxy-6-iodo-3-propyl-chromen-4-on, 3-(3-Brom-6-fluoro-2-methyl-indol-1-sulfonyl)-[1,2,4]triazol-1-sulfonsäuredimethylamid;

### Carbamate und Dithiocarbamate

- Dithiocarbamate: Ferbam, Mancozeb, Maneb, Metiram, Metam, Propineb, Thiram, Zineb, Ziram;
- Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethansulfonyl)-but-2-yl) carbaminsäure-(4-fluorphenyl)ester;

### Sonstige Fungizide

- Guanidine: Dodine, Iminoctadine, Guazatine;
- Antibiotika: Kasugamycin, Polyoxine, Streptomycin, Validamycin A;
- Organometallverbindungen: Fentin Salze;
- Schwefelhaltige Heterocyclylverbindungen: Isoprothiolane, Dithianon;
- Organophosphorverbindungen: Edifenphos, Fosetyl, Fosetyl-aluminium, Iprobenfos, Pyrazophos, Tolclofos-methyl, Phosphorige Säure und ihre Salze;
- Organochlorverbindungen: Thiophanate Methyl, Chlorothalonil, Dichlofluanid, Tolylfluanid, Flusulfamide, Phthalide, Hexachlorbenzene, Pencycuron, Quintozene;
- Nitrophenylderivate: Binapacryl, Dinocap, Dinobuton;
- Anorganische Wirkstoffe: Bordeaux Brühe, Kupferacetat, Kupferhydroxid, Kupferoxychlorid, basisches Kupfersulfat, Schwefel;
- Sonstige: Spiroxamine, Cyflufenamid, Cymoxanil, Metrafenone.

### Synthesebeispiele

1) Synthese von 1-Chlor-2-(4-chlor-2-fluorphenyl)-3-(2-chlorphenyl)propan-2-ol Zu einer Lösung von 2-Chlorbenzylchlorid (200 µl, 1.7 mmol) in wasserfreiem Diethylether (20 ml) wurden Magnesiumspäne (430 mg, 17.9 mmol) und lod gegeben. Das Reaktionsgemisch wurde langsam erwärmt, bis die lodfärbung verschwand und der Beginn der Reaktion durch Refluxieren des Lösungsmittels angezeigt wurde. Das restliche 2-Chlorbenzylchlorid (2.0 ml, 15.3 mmol) wurde tropfenweise so zugegeben, dass das Reaktionsgemisch refluxierte. Nach beendeter Zugabe wurde für weitere 2 h bei Raumtemperatur nachgerührt und anschließend auf 0 °C abgekühlt. Bei dieser Temperatur wurde 4-Chlor-2-fluorphenacetylchlorid (3.00 g, 14.5 mmol) in wasserfreiem Toluol (10 ml) tropfenweise zugegeben. Anschließend wurde auf Raumtemperatur erwärmt und für 3 h nachgerührt.. Nach dieser Zeit wurde wiederum auf 0 °C abgekühlt und mit einer gesättigtem wässrigen Ammoniumchlorid-Lösung (10 ml) versetzt. Die organische Phase wurde abgetrennt und die wässrige Phase mit Ethylacetat extrahiert (2x 20 ml). Die vereinigten organischen Extrakte wurden über Natriumsulfat getrocknet und vom Lösungsmittel befreit. Das so erhaltene Rohprodukt (5.0 g) wurde ohne Aufreinigung im nächsten Reaktionsschritt eingesetzt.
2) Synthese von (Z)-4-Chlor-1-[3-chlor-1-(2-chlorphenyl)prop-1-en-2-yl]-2-fluorbenzol Zu einer Lösung von 1-Chlor-2-(4-chlor-2-fluorphenyl)-3-(2-chlorphenyl)propan-2-ol (5.0 g, ca. 14.5 mmol) in einem 1,4-Dioxan/THF-Gemisch (44 ml, 10:1) wurden bei 0 °C zunächst Acetanhydrid (1.8 ml, 19.1 mmol) und anschließend konzentrierte Schwefelsäure (100 µl, 1.96 mmol) zugegeben. Das Reaktionsgemisch wurde anschließend auf Raumtemperatur erwärmt, für 18 h nachgerührt und anschließend wieder auf 0 °C gekühlt. Bei dieser Temperatur wurde gesättigte Kochsalzlösung zugegeben (20 ml) und mit wässriger Natriumhydroxid-Lösung (6.2 ml, 50 % w/w) neutralisiert. Das erhaltene Gemisch wurde mit Ethylacetat (3x 20 ml) extrahiert, die organischen Phasen vereinigt und über Natriumsulfat getrocknet. Nach Filtrieren und Entfernen des Lösungsmittels im Vakuum wurde der Rückstand säulenchromatographisch (Kieselgel, Hexan) aufgereinigt. Nach Vereinigung der entsprechenden Fraktionen wurde (Z)-4-Chlor-1-[3-chlor-1-(2-chlorphenyl)prop-1-en-2-yl]-2-fluorbenzol in Form eines farblosen Öles (1.40 g, 31 % über 2 Stufen) erhalten.
   ¹H NMR (300 MHz, CDCl₃) δ 8.01 (d, J = 6.9 Hz, 1H), 7.46-7.37 (m, 4H), 7.22-7.10 (m, 2H), 6.8 (s, 1H), 4.50 (s, 2H).
3) Herstellung von anti-2-(4-Chlor-2-fluorphenyl)-2-(chlormethyl)-3-(2-chlorphenyl)oxiran
   Zu einer Lösung von (Z)-4-Chlor-1-[3-chlor-1-(2-chlorphenyl)prop-1-en-2-yl]-2-fluorbenzol (800 mg, 2.53 mmol) in Essigsäure (20 ml) wurde Maleinsäureanhydrid (2.48 g, 25.3 mmol) und wässrige Wasserstoffperoxid-Lösung (1.46 ml einer 50 %igen Lösung, 25.3 mmol) hinzugegeben. Das Reaktionsgemisch wurde für 3 d bei 45 °C gerührt, anschließend auf Raumtemperatur abgekühlt und mit Wasser (20 ml) und wässriger Thiosulfat-Lösung (10 %ige Lösung, 4 ml) versetzt. Die wässrige Phase wurde mit Dichlormethan (3x 15 ml) extrahiert und die vereinigten organischen Phasen mit Kochsalzlösung (2x 10 ml) gewaschen. Die organische Phase wurde über Natriumsulfat getrocknet, abfiltriert und das Lösungsmittel abdestilliert. Der so erhaltene Rückstand wurde säulenchromatographisch (Kieselgel, 25:1 Hexan/Ethylacetat) aufgereinigt. Man erhielt anti-2-(4-Chlor-2-fluorphenyl)-2-(chlormethyl)-3-(2-chlorphenyl)oxiran (580 mg, 70 %) nach Vereinigen der entsprechenden Fraktionen in Form eines farblosen Feststoffes.
   ¹H NMR (300 MHz, CDCl₃) δ 7.50 (t, J = 9.0 Hz, 1H), 7.49-7.41 (m, 2H), 7.36-7.32 (m, 2H), 7.24-7.14 (m, 2H), 4.30 (s, 1H), 3.87 (d, J = 12.0 Hz, 1H), 3.37 (d, J = 12.0 Hz, 1H).
4) Herstellung von 1-[(anti)-2-(4-Chlor-2-fluorphenyl)-3-(2-chlorphenyl)oxiran-2-yl)methyl]-1H-1,2,4-triazol
   Zu einer Lösung von anti-2-(4-Chlor-2-fluorphenyl)-2-(chlormethyl)-3-(2-chlorphenyl)oxiran (580 mg, 1.74 mmol) in wasserfreiem N,N-Dimethylformamid (20 ml) wurde bei Raumtemperatur 1,2,4-Triazol (362 mg, 5.24 mmol) und Natriumhydrid (130 mg, 5.42 mmol) zugegeben. Das Gemisch wurde zunächst für 20 h bei 60 °C und für weitere 4 h bei 75 °C gerührt. Anschließend wurde auf Raumtemperatur abgekühlt, mit Ethylacetat (20 ml) verdünnt und mit Kochsalz-Lösung (3 x 15 ml) gewaschen. Die abgetrennte organische Phase wurde über Natriumsulfat getrocknet. filtriert und das Lösungsmittel abdestilliert. Der so erhaltene Rückstand wurde säulenchromatographisch (Kieselgel, 7:3 Hexan/Ethylacetat) aufgereinigt. Nach Vereinigen der entsprechenden Fraktionen wurde die Zielverbindung (383 mg, 60 %) in Form eines farblosen Feststoffes erhalten.
   ¹H NMR (300 MHz, CDCl₃) δ 7.86 (s, 1H), 7.78 (s, 1H), 7.61-7.58 (m, 1H), 7.57-7.37 (m, 3H), 7.23-7.06 (m, 3H), 4.76 (d, *J*= 12.0 Hz, 1H), 4.28 (s, 1H), 4.02 (d, *J*= 12.0 Hz, 1H).

Nach dieser Herstellvorschrift wurden die folgenden Verbindungen der allgemeinen Formel 1 als Racemate mit *cis*-Anordnung von Triazolylmethylsubstituent und Ring B der Tabelle 14 hergestellt. [*] = nicht vom Schutzumfang umfasst

**Tabelle 14**

| Verb.Nr. | A | B | Physikal. Daten FP [°C]. ¹H-NMR (300 MHz, CDC13) |
|---|---|---|---|
| 14.1 [*] | 3-Fluor-4-methoxyphenyl | 2-Chlorphenyl | 165-168 |
| 14.2 | 2-Chlor-4-fluorphenyl | 2-Fluorphenyl | 108-111 |
| 14.3 | 4-Chlor-2-fluorphenyl | 4-Chlorphenyl | 114-116 |
| 14.4 | 2-Chlor-4-fluorphenyl | 4-Fluorphenyl | 7.87 (1H),7.82 (1H), 7.58 (2H), 7.18 (3H), 7.08 (1H), 6.90 (1H), 4.70 (1H), 4.22 (1H), 4.03 (1H) |
| 14.5 | 2-Chlor-4-fluorphenyl | 2-Chlorphenyl | 92-97 |
| 14.6 [*] | 4-Chlor-2-fluorphenyl | 4-Methylphenyl | 91-93 |
| 14.7 | 4-Chlor-2-fluorphenyl | 2-Chlorphenyl | 121-123 |
| 14.8 | 2-Fluor-4-chlorphenyl | 3-Chlorphenyl | 7.89 (1H), 7.81 (1H), 7.53 (1H), 7.42 (3H), 7.12 (3H), 4.65 (1H), 4.20 (1H), 4.07 (1H) |
| 14.9 | 2-Fluor-4-chlorphenyl | 2-Fluorphenyl | 150-152 |
| 14.10 | 2-Fluor-4-methoxyphenyl | 2-Chlorphonyl | 115-116 |
| 14.11 [*] | 2-Fluor-4-methoxyphenyl | 2-Methylphenyl | 107-108 |
| 14.12 | 4-Fluor-2-methoxyphenyl | 2-Chlorphenyl | 112-115 |
| 14.13 | 4-Fluor-2-methoxyphenyl | 2-Fluorphenyl | 94-95 |
| 14.14 [*] | 4-Fluor-2-methoxyphenyl | 2-Methylphenyl | 104-106 |

### Biologische Versuche

### Gewächshaus

### Wirkstoffaufbereitung

Die Wirkstoffe wurden getrennt oder gemeinsam als eine Stammlösung aufbereitet mit 25 mg Wirkstoff, welcher mit einem Gemisch aus Aceton und/oder DMSO und dem Emulgator Wettol EM 31 (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegebenen Wirkstoffkonzentration verdünnt.

### Anwendungsbeispiel 1 - Wirksamkeit gegen die Dürrfleckenkrankheit der Tomate verursacht durch Altemaria solani (Alteso P1)

Blätter von getopften Tomatenpflanzen wurden mit einer wässriger Suspension in der unten angegebenen Wirkstoffkonzentration bis zur Tropfnässe besprüht. Am folgenden Tag wurden die Blätter mit einer wässrigen Sporensuspension von Altemaria solani in 2 % Biomalzlösung inokuliert. Anschließend wurden die Pflanzen in einer wasserdampf-gesättigten Kammer bei Temperaturen zwischen 20 und 22°C aufgestellt. Nach 5 Tagen hatte sich die Krankheit auf den unbehandelten, jedoch infizierten Kontrollpflanzen so stark entwickelt, dass der Befall visuell in % ermittelt werden konnte.

Die mit den Wirkstoffen 14.10, 14.12, 14.13 und 14.14 der Tabelle 14 mit 63 ppm-haltiger wässriger Wirkstoffaufbereitung behandelten Pflanzen zeigten einen Befall von max. 10 %, während die unbehandelten Pflanzen zu 90 % befallen waren.

### Mikrotest

Die Wirkstoffe wurden getrennt als Stammlösung formuliert mit einer Konzentration von 10000 ppm in DMSO.

### Anwendungsbeispiel Nr. 2 - Aktivität gegen den Verursacher der Grauschimmel Botrytis cinerea im Mikrotitter-Test (Botrci)

Die Stammlösung wird in eine Mikrotitterplatte (MTP) pipettiert und mit einem wässrigen Pilznährmedium auf Malzbasis auf die angegebene Wirkstoffkonzentration verdünnt. Anschließend erfolgte die Zugabe einer wässrigen Sporensuspension von Botrytis cinerea. Die Platten wurden in einer wasserdampf-gesättigten Kammer bei Temperaturen von 18°C aufgestellt. Mit einem Absorbtionsphotometer wurden die MTPs am 7. Tag nach der Inokulation bei 405nm gemessen.

Die gemessenen Parameter wurden mit dem Wachstum der wirkstofffreien Kontrollvariante und dem pilz- und wirkstofffreien Leerwert verrechnet, um das relative Wachstum in % der Pathogene in den einzelnen Wirkstoffen zu ermitteln.

Die mit den Wirkstoffen 14.2, 14.3, 14.4, 14.5, 14.6, 14.7, 14.8, 14.9 und 14.11 der Tabelle 14 mit 125 ppm-haltiger wässriger Wirkstoffaufbereitung behandelten Pathogene zeigten ein Wachstum von max. 2 %.

### Anwendungsbeispiel Nr. 3 - Aktivität gegen den Verursacher des Septoria Blattdürre Septoria tritici im Mikrotitter-Test (Septtr)

Die Stammlösung wird in eine Mikrotitterplatte (MTP) pipettiert und mit einem wässrigen Pilznährmedium auf Malzbasis auf die angegebene Wirkstoffkonzentration verdünnt. Anschließend erfolgte die Zugabe einer wässrigen Sporensuspension von Septoria tritici . Die Platten wurden in einer wasserdampf-gesättigten Kammer bei Temperaturen von 18°C aufgestellt. Mit einem Absorbtionsphotometer wurden die MTPs am 7. Tag nach der Inokulation bei 405nm gemessen.

Die gemessenen Parameter wurden mit dem Wachstum der wirkstofffreien Kontrollvariante (100 %)und dem pilz- und wirkstofffreien Leerwert verrechnet, um das relative Wachstum in % der Pathogene in den einzelnen Wirkstoffen zu ermitteln.

Die mit den Wirkstoffen 14.2, 14.3, 14.4, 14.5, 14.6, 14.7, 14.8, 14.9 und 14.11 der Tabelle 14 mit 125 ppm-haltiger wässriger Wirkstoffaufbereitung behandelten Pathogene zeigten ein Wachstum von max. 9 %.

### Vergleichsversuch

### Gewächshaus

### Wirkstoffaufbereitung

Die Wirkstoffe wurden getrennt oder gemeinsam als eine Stammlösung aufbereitet mit 25 mg Wirkstoff, welcher mit einem Gemisch aus Aceton und/oder DMSO und dem Emulgator Wettol EM 31 (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegeben Wirkstoffkonzentration verdünnt.

### Vergleichsbeispiel 1 - Wirksamkeit gegen die Dürrfleckenkrankheit der Tomate verursacht durch Altemaria solani (Alteso P1)

Blätter von getopften Tomatenpflanzen wurden mit einer wässriger Suspension in der unten angegebenen Wirkstoffkonzentration bis zur Tropfnässe besprüht. Am folgenden Tag wurden die Blätter mit einer wässrigen Sporensuspension von Altemaria solani in 2 % Biomalzlösung inokuliert. Anschließend wurden die Pflanzen in einer wasserdampf-gesättigten Kammer bei Temperaturen zwischen 20 und 22°C aufgestellt. Nach 5 Tagen hatte sich die Krankheit auf den unbehandelten, jedoch infizierten Kontrollpflanzen so stark entwickelt, dass der Befall visuell in % ermittelt werden konnte.

**Tabelle 15**

| Verb.-Nr.: | Struktur | Befall in % bei 63ppm bei Alteso P1 |
|---|---|---|
| 14.10 | 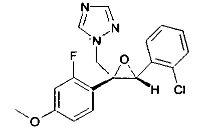 | 5 |
| Gemäß EP-A 0 196 038 | 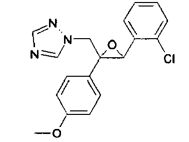 | 90 |

Die biologischen Ergebnisse der Tabelle 15 zeigen eine deutlich bessere fungizide Wirkung der erfindungsgemäßen Verbindung Nr. 14.10 im Vergleich zur strukturnächsten Verbindung des Standes der Technik gemäß EP-A 0 196 038.

## Patentansprüche

1. Azolylmethyloxirane der allgemeinen Formel I 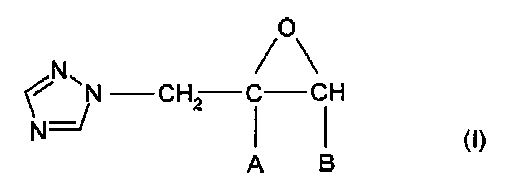 worin
A für Phenyl steht, das mit einem F und einem weiteren Substituenten substi- tuiert ist, ausgewählt aus Cl, C₁-C₄-Alkyl, C₁-C₄-Halogenalkyl und C₁-C₄- Alkoxy, wobei der der Phenylring in 2,4-Stellung substituiert ist,
B für Phenyl, das durch ein bis drei der folgenden Substituenten Halogen, NO₂, Amino, C₁-C₄-Alkoxy, C₁-C₄-Halogenalkyl, C₁-C₄-Halogenalkoxy, C₁- C₄-Alkylamino, C₁-C₄-Dialkylamino, Thio oder C₁-C₄-Alkylthio, substituiert ist,
sowie deren für Pflanzen verträgliche Säureadditions- oder Metallsalze.

2. Verbindungen nach Anspruch 1, bei denen A 2-Chlor-4-fluorphenyl, 2-Fluor-4-chlorphenyl, 2-Methoxy-4-fluorphenyl, 2-Fluor-4-methoxyphenyl oder 2-Fluor-4-methylphenyl, bedeutet.

3. Verbindungen nach Anspruch 1 oder 2, bei denen B für Phenyl steht, das durch ein bis drei der folgenden Substituenten Halogen, C₁-C₄-Alkoxy, C₁-C₄-Halogenalkyl oder C₁-C₄-Halogenalkoxy substituiert ist.

4. Verwendung von Verbindungen der Formel I gemäß einem der Ansprüche 1 bis 3 und ihrer Säureadditions- oder Metallsalze zur Bekämpfung von pflanzenpathogenen Pilzen.

5. Mittel für den Pflanzenschutz, enthaltend einen festen oder flüssigen Träger und eine Verbindung der Formel I gemäß einem der Ansprüche 1 bis 3 und/oder ein Säureadditions- oder Metallsalz davon.

6. Saatgut, enthaltend wenigstens eine Verbindung der Formel I gemäß einem der Ansprüche 1 bis 3 und/oder ein Säureadditions- oder Metallsalz davon.

7. Verfahren zur Bekämpfung von pflanzenpathogenen Pilzen, **dadurch gekennzeichnet, dass** man die Pilze, oder die vor Pilzbefall zu schützenden Materialien, Pflanzen, den Boden oder Saatgüter mit einer wirksamen Menge einer Verbindung der Formel I gemäß einem der Ansprüche 1 bis 3 oder einem Säureadditions- oder Metallsalz davon behandelt.

## Claims

1. An azolylmethyloxirane of the general formula I 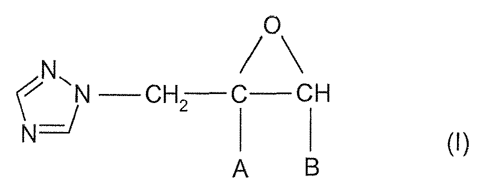 in which
A is phenyl which is substituted by an F and another substituent selected from the group consisting of Cl, C₁-C₄-alkyl, C₁-C₄- haloalkyl and C₁-C₄-alkoxy, the phenyl ring being substituted in the 2,4-position,
B is phenyl which is substituted by one to three of the following substituents: halo- gen, NO₂, amino, C₁-C₄-alkoxy, C₁-C₄- haloalkyl, C₁-C₄-haloalkoxy, C₁-C₄- alkylamino, C₁-C₄-dialkylamino, thio or C₁- C₄-alkylthio, or a plant-compatible acid addition salt or metal salt thereof.

2. The compound according to claim 1 in which A is 2-chloro-4-fluorophenyl, 2-fluoro-4-chlorophenyl, 2-methoxy-4-fluorophenyl, 2-fluoro-4-methoxyphenyl or 2-fluoro-4-methylphenyl.

3. The compound according to claim 1 or 2 in which B is phenyl which is substituted by one to three of the following substituents: halogen, C₁-C₄-alkoxy, C₁-C₄-haloalkyl or C₁-C₄-haloalkoxy.

4. The use of a compound of the formula I according to any of claims 1 to 3 or an acid addition salt or metal salt thereof for controlling phytopathogenic fungi.

5. A crop protection composition comprising a solid or liquid carrier and a compound of the formula I according to any of claims 1 to 3 and/or an acid addition salt or metal salt thereof.

6. Seed comprising at least one compound of the formula I according to any of claims 1 to 3 and/or an acid addition salt or metal salt thereof.

7. A method for controlling phytopathogenic fungi wherein the fungi or the materials, plants, the soil or seed to be protected against fungal attack are/is treated with an effective amount of a compound of the formula I according to any of claims 1 to 3 or an acid addition salt or metal salt thereof.

## Revendications

1. Azolylméthyloxiranes de formule générale I 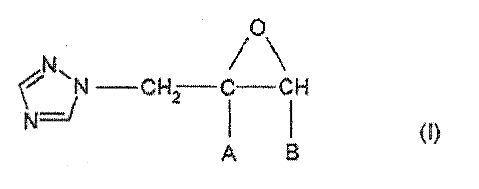 dans laquelle
A représente un phényle, qui est substitué avec un F et un autre substituant choisi parmi Cl, alkyle en C₁-C₄, halogénoalkyle en C₁-C₄ et alcoxy en C₁-C₄, le cycle phényle étant substitué en position 2,4,
B représente un phényle, qui est substitué par un à trois des substituants suivants : halogène, NO₂, amino, alcoxy en C₁-C₄, halogénoalkyle en C₁-C₄, halogénoalcoxy en C₁-C₄, alkylamino en C₁-C₄, dialkylamino en C₁-C₄, thio ou alkylthio en C₁-C₄,
ainsi que leurs sels d'addition acide ou métalliques compatibles avec les plantes.

2. Composés selon la revendication 1, dans lesquels A signifie 2-chloro-4-fluorophényle, 2-fluoro-4-chlorophényle, 2-méthoxy-4-fluorophényle, 2-fluoro-4-méthoxyphényle ou 2-fluoro-4-méthylphényle.

3. Composés selon la revendication 1 ou 2, dans lesquels B représente un phényle qui est substitué par un à trois des substituants suivants : halogène, alcoxy en C₁-C₄, halogénoalkyle en C₁-C₄ ou halogénoalcoxy en C₁-C₄.

4. Utilisation de composés de formule I selon l'une quelconque des revendications 1 à 3 et de leurs sels d'addition acide ou métalliques pour lutter contre les champignons pathogènes des plantes.

5. Agent pour la protection des plantes, contenant un véhicule solide ou liquide et un composé de formule I selon l'une quelconque des revendications 1 à 3 et/ou un de leurs sels d'addition acide ou métalliques.

6. Semence, contenant au moins un composé de formule I selon l'une quelconque des revendications 1 à 3 et/ou un de leurs sels d'addition acide ou métalliques.

7. Procédé de lutte contre les champignons pathogènes des plantes, **caractérisé en ce que** les champignons, ou les matériaux, les plantes, le sol ou les semences à protéger avant l'infestation par des champignons, sont traités avec une quantité efficace d'un composé de formule I selon l'une quelconque des revendications 1 à 3 ou d'un de leurs sels d'addition acide ou métalliques.
